# FASCICULE DE BREVET EUROPEEN

(11) **EP 2 399 132 B1**
(45) Date de publication et mention de la délivrance du brevet: **11.06.2014**
(21) Numéro de dépôt: 10708252.1
(22) Date de dépôt: 23.02.2010
(51) Int. Cl.: G01N 33/68, C12N 9/64, G01N 33/573

(54) **MÉTHODE D'ANALYSE PERMETTANT LE DIAGNOSTIC IN VITRO D'UNE HYPERPLASIE BÉNIGNE DE LA PROSTATE (HBP) CHEZ UN CHIEN PAR DOSAGE DE LA CANINE PROSTATE SPÉCIFIC ESTERASE ET LE SUIVI DU TRAITEMENT DE L'HBP**
ANALYSEVERFAHREN ZUR IN-VITRO-DIAGNOSE VON BPH (BENIGN PROSTATIC HYPERPLASIA) IN HUNDEN DURCH TESTEN DER HUNDEPROSTATA-SPEZIFISCHEN ESTERASE UND BPH-NACHSORGE
ANALYSIS METHOD FOR THE IN VITRO DIAGNOSIS OF BENIGN PROSTATIC HYPERPLASIA (BPH) IN DOGS BY ASSAYING CANINE PROSTATE SPECIFIC ESTERASE, AND BPH TREATMENT FOLLOW UP

(30) Priorité: 23.02.2009 FR 0900809
(43) Date de publication de la demande: 28.12.2011
(73) Titulaire: VIRBAC S.A., 06516 Carros (FR)
(72) Inventeur: CLARET, Emmanuel, F-30650 Rochefort du Gard (FR); AUDHUY, Stéphane, F-83500 La Seyne Sur Mer (FR); MORLET, Jérôme, 83210 Sollies Pont (FR); PAPIEROK, Gérard, F-83400 Hyères Les Palmiers (FR)
(74) Mandataire: Macquet, Christophe
(86) Numéro de dépôt international: PCT/FR2010/000172
(87) Numéro de publication internationale: WO 2010/094868

(56) Documents cités:
- WO-A2-01/50132
- BELL F W; ET AL: "EVALUATION OF SERUM AND SEMINAL PLASMA MARKERS IN THE DIAGNOSIS OF CANINE PROSTATIC DISORDERS" JOURNAL OF VETERINARY INTERNAL MEDICINE, LIPPINCOTT, PHILADELPHIA, US, vol. 9, no. 3, 1 mai 1995 (1995-05-01), pages 149-153, XP001014409 ISSN: 0891-6640 cité dans la demande
- ISAACS W B ET AL: "IMMUNOLOGICAL LOCALIZATION AND QUANTITATION OF THE ANDROGEN-DEPENDENT SECRETORY PROTEASE OF THE CANINE PROSTATE" ENDOCRINOLOGY, BALTIMORE, MD, US, vol. 117, no. 4, 1 janvier 1985 (1985-01-01), pages 1512-1520, XP001012294 ISSN: 0013-7227
- BVT: "Odelis CPSE - Présentation et avantages" online 2009, XP002544005 Extrait de l'Internet: URL:http://www.bvt.fr/p-bvtfrpubfr/display .aspx?srv=p-bvtfr&typ=pub&lang=fr&cmd=view &style=styles/specie.xsl&select=PRODUCT[@I D$eq$PRODUCT_119]|SECTION[@ID$eq$SECTION_2 45]&affp=&> [extrait le 2009-09-01]
- BVT: "Odelis CPSE - Protocoles" online 2009, XP002544006 Extrait de l'Internet: URL:http://www.bvt.fr/p-bvtfrpubfr/display .aspx?srv=p-bvtfr&typ=pub&lang=fr&cmd=view &style=styles/specie.xsl&select=PRODUCT[@I D$eq$PRODUCT_119]|SECTION[@ID$eq$SECTION_2 47]&affp=&> [extrait le 2009-09-01]
- GOBELLO CRISTINA; CASTEX GERVASIO; CORRADA YANINA: "Serum and seminal markers in the diagnosis of disorders of the genital tract of the dog: A mini-review" THERIOGENOLOGY, vol. 57, no. 4, 1 mars 2002 (2002-03-01), pages 1285-1291, XP002544004 ISSN: 0093-691X
- KLAUNSNER J S; JOHNSTON S D; BELL F W: "Canine prostatic disorders" 1 janvier 1995 (1995-01-01), JOURNAL OF VETERINARY INTERNAL MEDICINE, LIPPINCOTT, PHILADELPHIA, US, PAGE(S) 1103 - 1110 , XP008111550 ISSN: 0891-6640 le document en entier
- FRENETTE G; DUBÉ J Y; LACOSTE D; TREMBLAY R R: "Radioimmunoassay in blood plasma of arginine esterase: the major secretory product of dog prostate." THE PROSTATE 1987, vol. 10, no. 2, 1987, pages 145-152, XP002544003 ISSN: 0270-4137 cité dans la demande
- JOHNSTON S D ET AL: "Prostatic disorders in the dog.", ANIMAL REPRODUCTION SCIENCE 2 JUL 2000, vol. 60-61, 2 July 2000 (2000-07-02), pages 405-415, ISSN: 0378-4320
- K PACLIKOVA ET AL: 'Diagnostic possibilities in the management of canine prostatic disorders' VETERINARNI MEDICINA vol. 1, 01 Janvier 2006, pages 1 - 13, XP055077122

## Description

La présente invention a pour objet une méthode d'analyse permettant le diagnostic *in vitro* de l'hyperplasie bénigne de la prostate (HPB) chez un chien, en effectuant notamment le dosage du paramètre Canine Prostate Specific Esterase (CPSE) dans le sang telle que définie dans les revendications. Cette méthode permet de distinguer non seulement les HPB des prostates normalens mais également les HBP des affections plus sévères de la prostate canine comme les prostatites et les adénocarcinomes. L'invention est également appliquée au suivi de traitement des HPB.

### Art Antérieur :

L'hyperplasie bénigne de la prostate (HBP) est une affection très fréquente chez le chien adulte vieillissant : 50 à 80 % des chiens âgés de plus de cinq ans présentent à l'analyse histologique une hyperplasie et une hypertrophie prostatique (Johnston SD, Kustritz MVR, Olson PNS. Benign Prostatic Hypertrophy/Hyperplasia. In: Canine and feline theriogeology. Philadelphia: WB Saunders, 2001, 340-341). Dans la plupart des cas, l'HBP est sans répercussion pour le chien. Néanmoins, l'HBP peut être à l'origine de manifestations cliniques lorsqu'elle est importante (taille pouvant être 2 à 6 fois supérieure à la normale), et favoriser l'apparition d'autres affections prostatiques plus graves (prostatite, kystes volumineux, abcès prostatiques, etc.).

Il est donc essentiel pour le praticien de savoir diagnostiquer une HBP au plus tôt afin de prévenir le développement des lésions et l'apparition de symptômes marqués.

L'HBP est une affection chronique si le chien n'est pas castré : la prostate grossit progressivement s'il n'y a pas de traitement ou après l'arrêt du traitement. Afin d'éviter une aggravation ou une reprise des symptômes, il est important pour le vétérinaire de pouvoir suivre l'évolution de la prostate.

Les méthodes actuelles de diagnostic de l'HBP sont le toucher prostatique, la radiographie et l'échographie. Toutefois, ces trois méthodes n'apportent pas de résultats satisfaisants.

Le toucher prostatique est un examen qui présente de nombreuses limites liées notamment :
- à l'opérateur : il convient de palper de nombreuses prostates saines pour remarquer une HBP ;
- au chien : le toucher rectal est impossible chez les races de grandes tailles et miniatures. Il existe de plus des spécificités raciales à connaitre (Scottish terrier, etc.) ;
- à l'examen lui-même : la palpation se limite à la surface dorsale de la prostate ce qui implique que l'HBP doit être déjà à un stade avancé pour être diagnostiquée ;
- aux nombreux vétérinaires qui hésitent à pratiquer cet examen en présence du propriétaire.

L'examen radiographique présente plusieurs limites qui en font de nos jours un examen d'imagerie de second plan:
○ La radiographie surestime la taille de la prostate. Les organes avoisinants rendent la délimitation de la prostate difficile : colon, paroi abdominale, et du tissu adipeux péri-capsulaire (Feeney DA, Johnston GR, Klausner JS, et al : Canine prostatic disease-comparison of ultrasonographic appearance with morphologic and microbiologic findings : 30 cases (1981-1985). J Am Vet Med Assoc 1987 ;190 :1027-1034).
○ Une HBP est diagnostiquée quand le diamètre de la prostate est supérieur à 70% de la distance post-promontoire du sacrum - pointe du pubis. Toutefois, cette norme de 70 % utilisée en radiographie ne tient pas compte de l'âge du chien et de sa race. La prostate grossit physiologiquement avec l'âge du chien. La forme du bassin varie selon les races
○ L'évaluation du parenchyme prostatique est limitée : seule des calcifications ou des cavités de grandes tailles peuvent être remarquées.

Si l'échographie est l'examen de choix dans le diagnostic (et le dépistage) d'une HBP ainsi que dans le suivi du chien traité médicalement (récidives quasi-systématiques après plusieurs mois), elle semble parfois sous-diagnostiquer une HBP débutante ou sur-diagnostiquer une récidive.

L'étude de Ruel (Ruel Y, Barthez PY, Mailles A, Begon D : Ultrasonographic evaluation of the prostate in healthy intact dogs. Vet Radiol Ultrasound 1998 ; 39 :212-216) propose une fourchette large de la taille normale de la prostate. En effet, dans l'étude, 14 % des chiens considérés normaux, présentent un aspect glandulo-kystique du parenchyme prostatique (consécutif à une hyperplasie). Enfin, l'étude ne tient pas compte de l'influence de la race sur la taille de la prostate.

L'étude d'Atalan (Atalan G, Holt PE, Barr FJ et coll. Ultrasonographic estimation of prostatic size in canine cadavers. Res. In vet. Sci. 1999 ; 67 : 7-15) met l'accent sur les limites dans la fiabilité des mesures. Selon la technique échographique (position de la sonde, toucher rectal associé), de grandes variations des mesures sont possibles. La largeur (coupe transversale) souffre de la plus grande variabilité : une rotation de 30° de l'axe de la sonde en coupe transversale résulte en une variation allant jusqu'à 20 % de la mesure de la largeur de la prostate (Johnston SD, Kustritz MVR, Olson PNS. Benign Prostatic Hypertrophy/Hyperplasia. In: Canine and feline theriogeology. Philadelphia: WB Saunders, 2001, 340-341).

La difficulté des mesures s'explique notamment par :
- l'inclinaison de la sonde par rapport à l'axe de la prostate : présence du pénis (inclinaison latérale), prostate en partie engagée dans la filière pelvienne (inclinaison cranio-caudale) ;
- des contours prostatiques parfois difficiles à distinguer : qualité de l'échographe (fréquence de la sonde, etc.), prostate qui dépasse la fenêtre visuelle de la sonde ;
- une mauvaise position de la sonde sur la prostate : il faut prendre l'axe le plus grand. Un déplacement de la sonde latérale (coupe longitudinale) peut faire varier la mesure jusqu'à 10 %.

Ainsi, l'échographie souffre d'un manque de fiabilité (subjectivité de l'opérateur) dans la prise des mesures. De plus, le manque de reproductibilité des mesures rend le suivi de la taille de la prostate difficile au fil des mois : quand doit-on retraiter ?

Un marqueur biologique spécifique peut constituer une solution permettant de s'affranchir de ces problèmes de fiabilité et de reproductibilité tout en permettant d'obtenir un résultat objectif.

De tels marqueurs ont été recherchés dans l'urine de chien en comparant, par étude protéomique, des échantillons provenant de chiens sains et malades (Gemeiner M & Teinfalt M, Determination of changes in the prostate of dogs (WO/2001/050132)) mais il n'a pas été donné de suites à ces travaux.

La recherche de marqueurs à partir de liquide séminal a fait l'objet de nombreuses études.

A la différence de la plupart des espèces animales, le liquide séminal de chien contient un nombre très limité de protéines (Dubé JY, Frenette G, Chapdelaine P, Paquin R et Tremblay RR. Biochemical characteristics of the proteins secreted by dog prostate, a review. Exp Biol 1985 ; 43 : 149-159).

La phosphatase acide, un des marqueurs classiques de la prostate chez l'homme, est 100 fois moins concentrée dans le liquide séminal canin que chez l'homme (Dubé JY, Frenette G, Chapdelaine P, Paquin R et Tremblay RR. Biochemical characteristics of the proteins secreted by dog prostate, a review. Exp Biol 1985 ; 43 : 149-159 ); son activité enzymatique chez les chiens ne diffère pas significativement de celle des chiens atteints d'HBP (Bell FW, Klausner JS, Hayden DW et coll. Evaluation of serum and seminal plasma markers in the diagnosis of canine prostatic disorders. J Vet Intern Med. 1995 ; 9 :149-153).

En 1984, Chapdelaine et al. (Chapdelaine P, Dubé JY, Frenette G et coll. Identification of arginine esterase as the major androgen-dependent protein secreted by dog prostate and preliminary molecular characterization in seminal plasma. J Androl. 1984 ; 5 : 206-210) identifie le composant majoritaire du liquide séminal de chien, l'arginine estérase ou CPSE (Canine prostate specific antigen, SwissProt n° P09582). La CPSE appartient à la famille des kallikréines qui sont des protéases capables de synthétiser des kinines à partir de kininogène. Ces enzymes sont impliquées dans plusieurs processus physiologiques dont l'inflammation et la pression artérielle.

Un radioimmunoessai est développé (Frenette G, Dubé JY, Lacoste D & Tremblay RR Radioimmunoassay in blood plasma of arginine esterase : the major secretory product of dog prostate. The Prostate 1987 ; 10 : 145-152) permettant de doser la CPSE chez les chiens sains.

Un dosage de la CPSE chez des chiens sains et des chiens atteints d'HBP est réalisé par immunoprécipitation suivie d'un immunoblotting (Bell FW, Klausner JS, Hayden DW et coll. Evaluation of serum and seminal plasma markers in the diagnosis of canine prostatic disorders. J Vet Intern Med. 1995 ; 9 :149-153) mais les méthodes utilisées n'ont pas permis aux auteurs de distinguer, de manière statistiquement significative, les chiens atteints d'une affection prostatique d'une part, des chiens sains d'autre part. Les résultats donnés par cette étude ont une très faible spécificité et une très faible sensibilité. Par exemple, pour des chiens présentant des taux de CPSE inférieurs à 189 ng/ml, il n'est pas possible de déterminer avec fiabilité si ces chiens sont sains ou présentent une affection prostatique.

Les traitements actuellement proposés pour l'HBP sont la chirurgie ou la mise en place d'une hormonothérapie. Dans ce dernier cas, les molécules utilisées sont soit des antiandrogènes (stéroïdiens ou non-stéroïdiens), soit des agonistes de la LHRH (ou GnRH). Un suivi de l'action d'un agoniste de la LHRH chez le chien sain par le dosage de la CPSE sérique a été décrit (Frenette G, Dubé JY, Lacoste D & Tremblay RR Radioimmunoassay in blood plasma of arginine esterase : the major secretory product of dog prostate. The Prostate 1987 ; 10 : 145-152), mais ce suivi est généralement fait par évaluation de la taille de la prostate (échographie) et est donc soumis aux incertitudes liées à cette technique.

### Description de l'invention :

L'invention consiste en une méthode d'analyse permettant le diagnostic *in vitro* de l'HBP chez le chlen, en utilisant le taux ou la concentration de Canine Prostate Specific Esterase (CPSE) présent dans le sérum ou plasma comme marqueur de l'HBP telle que définie dans les revendications. Cette méthode utilise également des molécules biologiques fixant spécifiquement la CPSE telles que des anticorps polyclonaux et/ou monoclonaux spécifiques anti-CPSE, des récepteurs solubles ou des aptamères.

Plus le taux de CPSE présent dans les fluides biologiques du chien est important, plus l'HBP est développée. Toutefois, il existe un seuil ou valeur de référence en deçà duquel le chien peut, de manière suffisamment fiable pour que la méthode puisse être utilisée par les vétérinaires, être considéré comme non-HBP, et *a contrario* au dessus duquel le chien est déclaré comme présentant une HBP.

Par non-HBP, on entend un chien ne présentant pas d'HBP mais une affection autre de la prostate, telle que la prostatite et/ou l'adénocarcinome de la prostate, ou encore, un chien sain.

L'invention permet des résultats fiables, en particuller chez le chien ayant un taux sanguin de CPSE inférieur à 189 ng/ml.

Plus particulièrement, invention concerne une méthode d'analyse permettant le diagnostic *in vitro* d'une HBP chez un chien, en utilisant la CPSE contenue dans des échantillons de sérum ou de plasma prélevés sur la chien ainsi que des molécules biologiques fixant spécifiquement la CPSE, caractérisée en ce que:
a. on prélève un échantillon de sang chez un chien ;
b. on mesure une concentration en Canine Prostate Specific Esterase (CPSE) dudit échantillon de sérum ou de plasma ;
c. on compare ladite concentration mesurée en CPSE à une valeur de référence ; cette valeur étant comprise entre 34,8 ng/ml et 102,4 ng/ml pour le sérum ou le plasma, de préférence environ 61 ng/ml
d. on utilise la concentration en CPSE comme marqueur de l'HBP; et
e. on diagnostique l'HBP chez le chien pour des concentrations de CPSE mesurées dans lesdits échantillons supérieures à ladite valeur de référence.

Ainsi, de façon avantageuse, cette méthode permet de diagnostiquer une hyperplasie prostatique bénigne chez le chien tout en la distinguant de la prostatite et/ou de l'adénocarcinome prostatique ainsi que de la prostate normale.

L'invention consiste également en une méthode d'analyse selon l'invention permettant de suivre le traitement thérapeutique d'une HBP, c'est-à-dire de suivre l'involution de la prostate lors d'un traitement avec des anti-androgènes, et d'adapter en fonction le traitement de l'animal (= permet de répondre à la question : quand doit-on retraiter ?).

L'invention concerne également l'utilisation d'un kit de diagnostic *in vitro* comprenant des molécules biologiques fixant spécifiquement la CPSE pour doser la CPSE contenue dans le sérum ou le plasma du chien destiné à diagnostiquer une HBP chez le chien ayant un taux sérique ou plasmatique de CPSE superieur à une valeur comprise entre 34,8 ng/ml et 102,4 ng/ml, de préférence compris entre environ 61 ng/ml et environ 102,4 ng/ml, ou de suivre le traitement thérapeutique d'un chien atteint d'une HBP.

Selon un premier mode de réalisation de l'invention, on utilise la concentration de CPSE contenue dans les fluides biologiques prélevés sur le chien (sérum, plasma) comme marqueur de l'HBP. Les analyses et études conduites par les inventeurs et exposées ci-après ont permis de définir un seuil ou valeur de référence de cette concentration. Ainsi, un chien est considéré comme présentant une HBP dès lors que le taux sérique ou plasmatique de CPSE présent dans le fluide biologique est supérieur à 34 ng/ml, et de préférence égal à environ 01ng/ml.

Les analyses statistiques ont permis de démontrer que le seuil de 61 ng/ml de CPSE est un seuil clinique optimal. Cette concentration correspond à une sensibilité du test de 97,1% et une spécificité de 92,7%. Une variabilité autour de cette valeur de seuil clinique sera admise de sorte que la sensibilité ou la spécificité soient de préférence supérieures à 80%, préférentiellement supérieures à 90% et encore plus préférentiellement supérieures à 95%.

Toutefois, il a été considéré qu'une méthode d'analyse serait acceptable pour les praticiens si elle présentait une sensibilité et une spécificité proches de ou supérieures à 80%. Ainsi, la concentration en CPSE dans les fluides biologiques du chien peut être utilisée comme marqueur de l'HBP lorsque sont considérés comme présentant de manière fiable une HBP les chiens présentant un taux de CPSE suffisant pour que l'analyse statistique des données permette une sensibilité et une spécificité du test supérieures à 80%.

Comme présenté dans le tableau 9 ci-dessous, dans la méthode de diagnostic selon l'invention, les valeurs de références comprises entre 34 ng/ml et 102,4 ng/ml sont utilisables aussi bien lors de l'utilisation d'échantillon de sérum que de plasma.

Pour ce qui concerne des échantillons de sang, dont le plasma représente environ 55% du volume total, les valeurs de références pourront être comprises entre environ 18 ng/ml et environ 56 ng/ml lorsque, dans la méthode de diagnostic selon ce document on utilise des échantillons de sang.

Des valeurs de références correspondantes dans l'urine, dans le liquide séminal ou dans tout autre fluide biologique, proportionnelles aux valeurs ci-dessous peuvent être déterminées par l'homme du métier par des expériences de routine à sa portée.

Selon l'invention, la valeur de référence est préférentiellement égale à 61 ng/ml pour des échantillons de sérum ou de plasma.

Avantageusement, la méthode d'analyse selon l'invention comprend une étape additionnelle selon laquelle l'HBP est distinguée de la prostatite et/ou de l'adénocarcinome de la prostate. De façon préférée, ladite méthode permet de distinguer l'HBP de la prostatite et de l'adénocarcinome de la prostate.

De façon avantageuse encore, lorsqu'un chien étant traité par un agent anti-androgène, la méthode selon l'invention permet de déterminer l'efficacité dudit traitement au moyen de mesures de la concentration en CPSE effectuées à intervalles réguliers.

Un procédé utilisant une méthode d'analyse selon ce document est aussi décrit. Ce procédé permet avantageusement de suivre le traitement thérapeutique d'une HBP.

Ce procédé consiste en les étapes suivantes :
- mesurer la concentration en CPSE dans un échantillon prélevé à partir de fluides biologiques du chien
- diagnostiquer une HBP en comparant la valeur de cette concentration aux valeurs de référence
- traiter l'animal par un anti-androgène selon le protocole indiqué en cas d'HBP par le fabricant de cet anti-androgène
- effectuer à intervalles réguliers après traitement des mesures de la concentration en CPSE dans les fluides biologiques de l'animal.

L'invention concerne également l'utilisation d'un kit de diagnostic *in vitro* comprenant des molécules biologiques fixant spécifiquement la CPSE pour doser la CPSE contenue dans le sérum ou le plasma du chien destiné à diagnostiquer une HBP chez le chien ayant un taux sérique de CPSE superieur à une valeur comprise entre 34,8 ng/ml et 102,4 ng/ml, de préférence compris entre 61 ng/ml et 102,4 ng/ml, ou de suivre le traitement thérapeutique d'un chien atteint d'une HBP. Les molécules biologiques fixant spécifiquement la CPSE sont avantageusement des anticorps polyclonaux ou monoclonaux, des récepteurs solubles ou des aptamères.

Lesdites molécules biologiques fixant spécifiquement la CPSE sont avantageusement couplées à des grosses molécules de type blotine, avidine, streptavidine.

De façon préférée, les molécules biologiques fixant spécifiquement la CPSE sont immobilisées à un support solide, tel que notamment des membranes de nitrocellulose ou autres polymères, des supports latex, ou des matériels de plastique.

Les molécules biologiques fixant spécifiquement la CPSE ainsi que la CPSE sont conjugués à des radio isotopes, des molécules fluorescentes, des molécules luminescentes, des enzymes, des particules de latex, des colloïdes métalliques ou des particules magnétiques.

### • Expériences réalisées :

Afin de satisfaire leur étude, les inventeurs ont conduit leur analyse à partir du liquide séminal et du sérum prostatique prélevé sur le chien, sans que cela ait un caractère limitatif. Il est entendu que la méthode selon ce document peut s'appliquer à des échantillons prélevés à partir d'autres fluides biologiques tels que l'urine, le plasma sanguin, le liquide lacrymal, et les sécrétions salivaires.

### Purification de la CPSE :

La CPSE est purifiée à partir de liquide prostatique comme décrit dans le document Isaacs WB (Isaacs WB , Shaper JH. Isolation and characterisation of the major androgen-dependent glycoprotein of canine prostatic fluid. 1983 J. Biol. Chem. 258 : 6610-6615).

### Préparation de l'antisérum anti-CPSE :

Le sérum polyclonal de lapin anti-CPSE est préparé en immunisant des lapins New Zealand White contre de la CPSE purifiée. Les animaux reçoivent 5 injections de 300µg d'antigène chacune, à J0, J14, J28, J42 et J56. Les lapins sont sacrifiés 77 jours après la première injection. Les anticorps anti-CPSE sont purifiés à partir du sérum sur une colonne de protéine A.

### Immunoréactivité de l'anti-sérum analysée par western blot :

Du liquide séminal ou du sang d'un chien atteint d'HBP sont prélevés, le sérum étant obtenu par centrifugation du sang. Quatre microlitres de chacun de ces liquides biologiques sont séparés par électrophorèse de type SDS-PAGE en conditions réductrices ou non réductrices.

On réalise ensuite un électrotransfert de ce gel sur une membrane de nitrocellulose. Celle-ci est ensuite séchée puis saturée.

Une immuno-empreinte est effectuée sur cette membrane : on l'immerge dans une dilution d'anticorps anti-CPSE pendant une heure, puis on révèle avec un anticorps anti-espèce couplé à la phosphatase alcaline. L'étape finale de révélation est réalisée avec du (3-bromo-4-chloro-5-indolyl phosphate et du nitro blue tetrazolium) BCIP/NBT.

### Préparation d'anticorps monoclonaux anti-CPSE :

Cinq souris Balb/c ont été injectées avec de la CPSE purifiée. Le titre sérique en anti-CPSE a été suivi par ELISA. Lorsque ce titre a atteint un niveau suffisant, les splénocytes ont été récupérés et fusionnés avec des cellules de myélomes murins SP2O. Après une succession de clonages et de sélections, les clones produisant un anticorps monoclonal anti-CPSE ont été sélectionnés et amplifiés.

### Détermination de la concentration en CPSE par sandwich ELISA :

La concentration en CPSE des échantillons sériques ou plasmatiques a été déterminée par ELISA de type sandwich. Des microplaques 96 puits (Stripwell Costar/Corning, NY, USA) sont recouvert d'anticorps anti-CPSE monoclonal ou polyclonal, à la concentration de 5µg/mL en tampon Carbonate 50 mM pH 9,6 une nuit à +4°C. Après 4 lavages, les puits sont incubés une heure à 37°C avec du tampon phosphate salin pH 7,2 additionné de 5% de sérum albumine bovine. Finalement les plaques sont incubées 30 minutes à température ambiante avec du tampon phosphate salin pH 7,2 additionné de 10% de saccharose. Une fois vidées, les microplaques sont séchées une nuit sous vide, puis ensachées en présence de dessicant jusqu'à utilisation.

Le dosage des échantillons se fait en distribuant 100µl de sérum ou de plasma (plasma héparine ou plasma EDTA) dans chacun des puits. Après une heure à 37°C, la microplaque est lavée. 100µl d'anticorps anti-CPSE monoclonal ou polyclonal conjugué à la peroxidase sont distribués. Les puits sont incubés 1 h à 37°C et de nouveau lavés. Les anticorps restés fixés sur les parois sont révélés par l'addition de 3,3',5,5' tetramethyl benzidine. Après arrêt de la réaction l'absorbance est lue à 450nm.

L'ELISA a été calibré avec de la CPSE purifiée. Le dosage présente une réponse linéaire pour des concentrations de CPSE comprises entre 0,39 et 20 ng/ml.

### Détermination du taux de CPSE par Immunochromatographie sur membrane

La CPSE sérique a été mesurée en utilisant la méthode décrite dans la demande de brevet WO03062824. Un premier anticorps anti-CPSE monoclonal ou polyclonal est immobilisé sur une membrane de nitrocellulose. Le sérum à quantifier est mélangé à un second anticorps anti-CPSE monoclonal ou polyclonal conjugué à un fluorophore, ce mélange est déposé sur la membrane de nitrocellulose. Lors de la migration, le complexe CPSE anticorps fluorophore est fixé par l'anti-CPSE immobilisé sur la membrane. La quantité d'anti-CPSE fluorescent fixée est proportionnelle à la quantité de CPSE contenue dans l'échantillon. La mesure de la fluorescence émise permet ainsi de quantifier la CPSE.

### Analyse statistique des données :

L'échantillon étudié inclut 89 chiens dont les caractéristiques sont décrites dans le tableau 1 ci-après :

| | |
|---|---|
| Taille de l'échantillon d'étude | 89 chiens |
| Chiens SANS signe clinique et/ou échographique d'HBP | 48 chiens |
| Chiens avec adénocarcinome | 2 chiens |
| Chiens avec Prostatite | 5 chiens |
| Chiens AVEC signes cliniques et/ou échographiques d'HBP | 34 chiens |

et détaillées dans le tableau 2 ci-après :

| **Nom** | **âge** | **Statut** |
|---|---|---|
| L9 | | HBP |
| L7 | | Adénocarcinome |
| D1 | 10 ans | Prostatite |
| D3 | | Prostatite |
| D8 | 18 mois | Normal |
| BM3 | 8 mois | Normal |
| BM12 | 9 mois | Normal |
| BM28 | 11 mois | Normal |
| BM29 | 11.5 mois | Normal |
| BM7 | 8 mois | Normal |
| G4 | 10 mois | Normal |
| BM14 | 8 mois | Normal |
| BM1 | 14 mois | Normal |
| BM5 | 20 mois | Normal |
| BM8 | 9 mois | Normal |
| BM11 | 9 mois | Normal |
| BM30 | 11.5 mois | Normal |
| BM25 | 11 mois | Normal |
| BM23 | 11 mois | Normal |
| BM17 | 13.5 mois | Normal |
| BM20 | 12.5 mois | Normal |
| D16 | 8 ans | Prostatite |
| BM19 | 12 mois | Normal |
| BM16 | 13 mois | Normal |
| BM18 | 12 mois | Normal |
| D9 | <24 mois | Normal |
| G1 | 17,5 mois | Normal |
| G3 | 17 mois | Normal |
| BM21 | 12.5 mois | Normal |
| BM10 | 17 mois | Normal |
| V3 | 14 mois | Normal |
| G2 | 17,5 mois | Normal |
| BM9 | 18 mois | Normal |
| BM27 | 11 mois | Normal |
| BM2 | 8 mois | Normal |
| BM13 | 9 mois | Normal |
| V5 | 14 mois | Normal |
| BM26 | 11 mois | Normal |
| BM22 | 11 mois | Normal |
| M15 | 2 ans | Normal |
| BM24 | 11 mois | Normal |
| BM6 | 5 mois | Normal |
| L17 | | Prostatite |
| D5 | 2 ans 9m | Normal |
| V2 | 14 mois | Normal |
| V6 | 14 mois | Normal |
| D10 | <24 mois | Normal |
| V7 | 19 mois | Normal |
| L24 | | Normal |
| BM15 | 16 mois | Normal |
| V1 | 14 mois | Normal |
| V4 | 14 mois | Normal |
| M24 | | HBP |
| M4 | 9 ans | HBP |
| L23 | | HBP |
| L15 | | HBP |
| D15 | <2ans | Normal |
| D19 | 15.5 ans | HBP |
| D2 | 13 ans | Adénocarcinome |
| M7 | | HBP |
| D11 | 13.5 ans | HBP |
| L3 | | HBP |
| L3 | | HBP |
| M5 | 12 ans | HBP |
| M22 | | HBP |
| L1 | | HBP |
| L19 | | HBP |
| L4 | | Prostatite |
| P4 | | HBP |
| D12 | 9 ans | HBP |
| M3 | | HBP |
| D4 | 4,8 ans | Normal |
| D6 | 10 ans | HBP |
| P6 | | HBP |
| D21 | 12 ans | HBP |
| D18 | 10 ans | HBP |
| D17 | 8.5 ans | HBP |
| M25 | | HBP |
| M20 | | HBP |
| L18 | | HBP |
| M26 | | HBP |
| L5 | | HBP |
| P7 | | HBP |
| L22 | | HBP |
| M6 | | HBP |
| M33 | | HBP |
| M1 | | HBP |
| D2 | 7 ans | HBP |
| M2 | | HBP |
| D7 | | HBP |

Pour ces chiens, des échantillons sériques ont été prélevés, aliquotés et congelés à -80°C jusqu'à utilisation.

Sont considérés comme étant potentiellement atteints d'HBP les chiens présentant les symptômes prostatiques suivants :
- pertes de sang par goutte au fourreau entre les mictions (d'origine prostatique)
- hématurie
- constipation
- boiterie des postérieurs
- hématospermie
- Incontinence

Cette constatation de symptômes peut éventuellement être confirmée par des examens complémentaires, des échographies et, pour certains, un examen histologique en complément de l'échographie.

Les chiens normaux sont considérés comme étant ceux présentant une échographie prostatique normale.

### Suivi de traitement avec des anti-androgènes :

5 chiens présentant des signes d'HBP (Voir critères d'inclusions décrits ci-dessus) ont été traités avec de l'acétate d'osatérone (YPOZANE^{®}, Virbac). Cette molécule est un anti-androgène qui agit sur le parenchyme prostatique. Le taux de CPSE a été mesuré à J0 et J+15 jours. Les chiens ont suivi le traitement conseillé et ont absorbé de 0,25 à 0,5 mg/kg/jour d'acétate d'osatérone, ceci pendant 7 jours. Du sérum a été prélevé avant tout traitement puis après 15 jours. Le taux de CPSE a été mesuré dans les différents échantillons.

### Analyse statistique des données

Les concentrations en CPSE sériques chez les chiens ayant été diagnostiqués comme ayant une HBP ont été comparées aux concentrations chez les chiens n'ayant pas d'HPB par un test de Wilcoxon. Une analyse de variance non paramétrique sur les rangs a été lancée afin de comparer les valeurs de concentration des groupes Adénocarcinome, Normal et Prostatite au groupe de chiens HBP. Comme il s'agit d'un test de comparaisons multiples, la valeur de p a été ajustée par l'ajustement de Dunnett. Les valeurs de p supérieures à 0,01 sont considérées comme non significatives.

Afin de vérifier la spécificité des anticorps de lapin anti-CPSE purifiés, quatre microlitres de sérum ou de liquide séminal ont été déposés sur un gel d'acrylamide contenant du SDS, et dans des conditions réductrices ou non réductrices. Après migration, les protéines sont transférées sur une membrane de nitrocellulose. Une fois saturée, cette membrane est incubée en présence des anticorps de lapin anti-CPSE purifiés. Après lavage, les anticorps restés fixés sont mis en évidence par des anticorps de chèvre anti-lapin couplés à la phosphatase alcaline. La phosphatase alcaline est révélée grâce à un substrat chromogénique le BCIP/NBT.

Les figures ci-jointes illustrent les résultats obtenus et sont données à titre d'exemples explicatifs pour aider à la compréhension de l'invention. Elles n'ont pas de caractère limitatif.

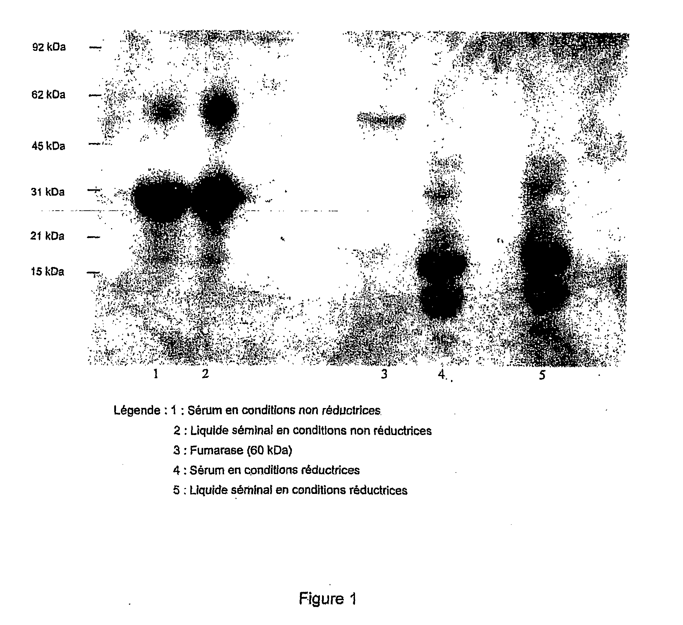

La figure 1 représente la réactivité de l'immunosérum contre du liquide prostatique ou du sérum de chiens présentant une HBP (Légende : 1 : Sérum en conditions non réductrices ; 2 : Liquide séminal en conditions non réductrices ; 3 : Fumarase (60 kDa) ; 4 : Sérum en conditions réductrices ; 5 : Liquide séminal en conditions réductrices).

Cette figure montre en pistes 1 et 2 une bande majeure à 30 kDa correspondant à la forme non réduite de la CPSE. Une bande à 60 kDa est aussi observée, elle correspond à un oligomère de la CPSE (Isaac WB et Sharper HS Immunological localisation and quantification of the androgen-dependent secretory protease of the canine prostate. 1985 Endocrinology 117 : 1512-1520). Lorsque les protéines sont réduites, pistes 4 et 5, nous observons bien les deux chaînes 15 kDa (chaîne H) et 12-14 kDa (chaîne L) de la CPSE.

Nous en concluons que les anticorps de lapins purifiés sont bien dirigés contre la CPSE et ne reconnaissent pas d'autres protéines sériques ou contenues dans le liquide séminal.

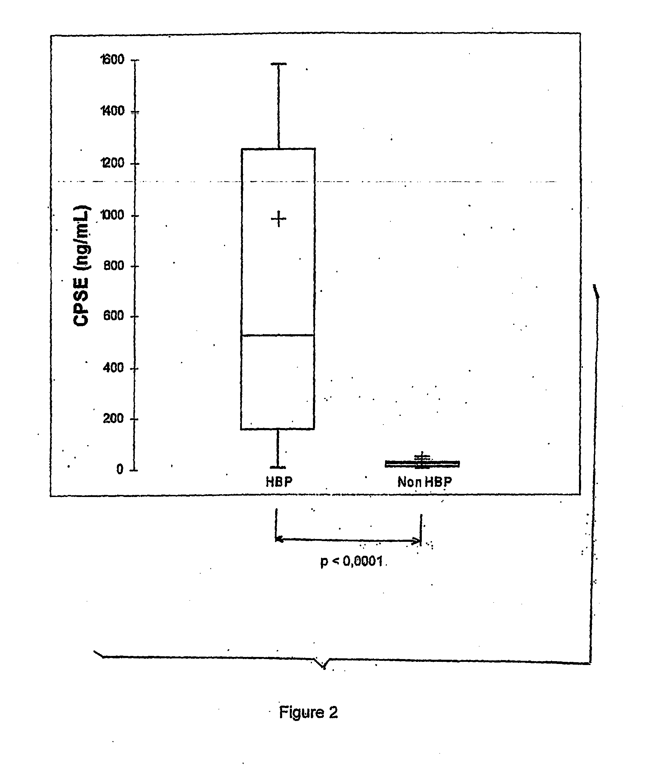

La Figure 2 montre les concentrations en CPSE sérique chez des chiens présentant des signes cliniques d'HBP en comparaison de ceux n'en présentant pas. Ces mesures ont été obtenues par le dosage ELISA décrit dans les paragraphes précédents.

Les data sont représentées par un diagramme en boite à moustaches. (+) correspond à la moyenne, (-) correspond à la médiane de l'échantillon. La limite inférieure du rectangle correspond au premier quartile, la limite supérieure au troisième quartile. Les extrémités des segments représentent les premier et neuvième déciles.

L'analyse statistique des données par un test de Wilcoxon, donne un p < 0,0001 montrant que les concentrations en CPSE dans le groupe de chiens présentant des signes cliniques d'HBP sont significativement plus élevées que dans le groupe de chiens n'en présentant pas.

**34 chiens sont diagnostiqués avec une HBP, 55 sont diagnostiqués non HBP.**

| **Variable analysée : CPSE (ng/ml)** | | | | | | |
|---|---|---|---|---|---|---|
| | **N** | **Moyenne** | **Ecart type** | **Médiane** | **Minimum** | **Maximum** |
| HBP | 34 | 981.51 | 1282.01 | 527.05 | 5.70 | 6047.00 |
| Non HBP | 55 | 38.79 | 82.31 | 22.00 | 5.90 | 507.70 |

Cette analyse statistique illustrée au tableau 3 ci-dessus démontre que la méthode selon l'invention est fiable et permet une bonne distinction des chiens présentant véritablement une HBP des chiens non-HBP.

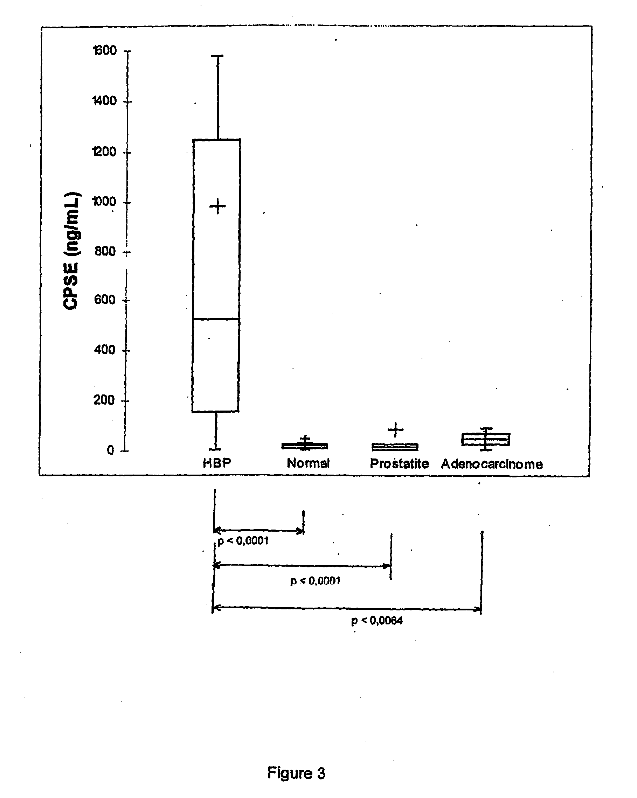

La Figure 3 montre les concentrations sériques en CPSE chez des chiens présentant une HBP comparé aux taux de CPSE chez des chiens présentant d'autres pathologies de la prostate (prostatite, adénocarcinome) ou des chiens normaux en bonne santé. Ces mesures ont été obtenues par le dosage ELISA décrit dans le paragraphe matériels et méthodes.

Les concentrations en CPSE dans les différents groupes ont été comparées par une analyse de variance non paramétrique sur les rangs. Les valeurs de p ont été ajustées par l'ajustement de Dunnett.

Les résultats (HBP vs Adénocarcinome p<0.0064 ; HBP vs Prostatite p< 0,0001 ; HBP vs Normaux p< 0,0001) indiquent que les taux de CPSE sériques chez les chiens présentant un adénocarcinome, une prostatite ou chez les chiens normaux sont significativement inférieurs aux taux de CPSE chez les chiens présentant une HBP.

Cette analyse statistique démontre que la méthode selon l'invention est fiable et permet une bonne distinction des chiens présentant une HBP de ceux présentant une prostatite ou un adénocarcinome, ou des chiens sains.

48 chiens ont une prostate normale (n=48), 34 chiens présentent des signes cliniques d'une HBP (n=34), 5 chiens présentent des signes de prostatite (n=5) et 2 chiens ont été diagnostiqués comme présentant un adénocarcinome (n=2). Voir tableau 4 ci-dessous :

| **Variable analysée : CPSE (ng/ml)** | | | | | | |
|---|---|---|---|---|---|---|
| | **N** | **Moyenne** | **Ecart type** | **Médiane** | **Minimum** | **Maximum** |
| Adénocarcinome | 2 | 48.15 | 59.75 | 48.15 | 5.90 | 90.40 |
| HBP | 34 | 981.51 | 1282.01 | 527.05 | 5.70 | 6047.00 |
| Normal | 48 | 33.25 | 71.32 | 22.40 | 6.50 | 507.70 |
| Prostatite | 5 | 88.16 | 165.36 | 15.60 | 6.40 | 383.50 |

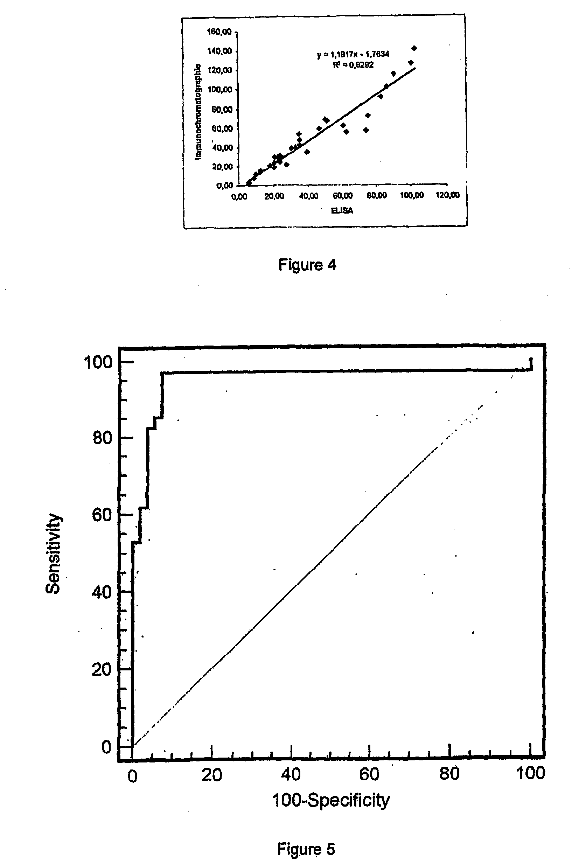

La figure 4 est une corrélation entre la mesure de la CPSE par ELISA et par immunochromatographie sur membrane.

Le taux de CPSE sérique a été mesuré soit en utilisant la méthode ELISA soit par Immunochromatographie quantitative sur membrane décrite dans les paragraphes précédents. Les concentrations mesurées dans les deux systèmes sont représentées dans cette figure. La valeur de r² = 0,92 montre que les deux méthodes sont bien corrélées.

La figure 5 est une analyse ROC (Receiver operating characteristic) de l'échantillon de chiens.

Afin de déterminer le taux de CPSE sérique permettant de diagnostiquer avec la plus grande confiance possible une HBP, les inventeurs ont analysé un échantillon de 89 chiens pour lesquels toutes les données cliniques et échographiques étaient connues.

L'analyse ROC (Receiver operating characteristic) de cet échantillon avec un seuil de décision clinique de 61 ng/ml permet de diagnostiquer l'HBP avec une sensibilité de 97,1% et une spécificité de 92,7%. L'indice de younden (sensibilité + spécificité-1) dans ces conditions est égal à 0,898, montrant ainsi la pertinence du seuil.

Toutefois, une sensibilité ou une spécificité de 80% représentent des niveaux acceptables de confiance dans le diagnostique. Dans le tableau ci-après, les inventeurs ont réitéré l'analyse ROC en utilisant différents seuils de décision cliniques. Il apparaît qu'il est possible de diagnostiquer une HBP avec une sensibilité de 79 % en utilisant un seuil de 102,4 ng/ml.

Inversement, le diagnostic d'HBP peut-être obtenu avec une spécificité de 80% en prenant comme seuil de décision clinique le taux de CPSE sérique de 34,8 ng/ml. Voir tableau 5 ci-dessous :

| | Sensibilité | Spécificité | Taux de CPSE sérique ou plasmatique |
|---|---|---|---|
| Décroissance de la sensibilité | 97,1% | 92,7% | 61 ng/ml |
| | 94% | 93% | 62,8 ng/ml |
| | 88% | 93% | 75,4 ng/ml |
| | 85% | 93% | 82,6 ng/ml |
| | 80% | 96% | 102,4 ng/ml |
| Décroissance de la spécificité | 97% | 89% | 50 ng/ml |
| | 97% | 86% | 39,3 ng/ml |
| | 97% | 80% | 34,8 ng/ml |

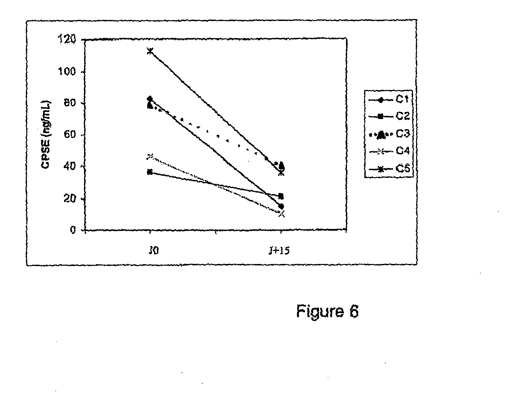

La figure 6 montre l'évolution du taux de CPSE sérique lors d'un suivi de traitement par un anti-androgène.

Des chiens présentant des signes cliniques d'HBP ont été traités avec de l'acétate d'osatérone, comme recommandé par le fabriquant. Le taux sérique de CPSE chez ces chiens a été mesuré avant tout traitement et après 15 jours. Voir tableau 6 à 8 ci-dessous :

**Tableau 6 :**

| N° cas | date 1^{er} prélèvement | date 2^{éme} prélèvement |
|---|---|---|
| 1 | 08/01/2009 | 23/01/2009 |
| 2 | 19/01/2009 | 03/02/2009 |
| 3 | 19/01/2009 | 03/02/2009 |
| 4 | 19/01/2009 | 03/02/2009 |
| 5 | 19/01/2009 | 03/02/2009 |

**Tableau 7 :**

| | CPSE (ng/ml) | |
|---|---|---|
| | Visite 1 | Visite 2 |
| C1 | 83 | 15 |
| C2 | 36 | 21 |
| C3 | 79 | 40 |
| C4 | 46 | 10 |
| C5 | 113 | 36 |

**Tableau 8 :**

| | CPSE (ng/ml) | |
|---|---|---|
| | J0 | J + 15 |
| Chien 1 | 83 | 15 |
| Chien 2 | 36 | 21 |
| Chien 3 | 79 | 40 |
| Chien 4 | 46 | 10 |
| Chien 5 | 113 | 36 |

Les résultats montrent une diminution du taux de CPSE sérique entre le moment où le traitement avec l'acétate d'osatérone n'a pas encore commencé, et le moment où le traitement est terminé. En parallèle, il a été observé un retour à la taille normale de la prostate chez tous ces chiens, ceci par toucher rectal.

Le taux sérique de CPSE peut donc être utilisé pour suivre un traitement de l'HBP par un anti androgène. Ce taux est donc un indicateur de l'efficacité du traitement.

**Tableau 9 : comparaison d'échantillon de sérum et de plasma**

| | CPSE (ng/ml) | |
|---|---|---|
| Echantillon | Sérum | Plasma héparinisé |
| 1 | 36 | 37 |
| 2 | 44 | 48 |
| 3 | 33 | 33 |
| 4 | 48 | 53 |
| 5 | 31 | 30 |
| 6 | 60 | 60 |
| 7 | 16 | 16 |
| 8 | 35 | 38 |
| 9 | 42 | 46 |
| 10 | 29 | 29 |
| 11 | 18 | 19 |
| 12 | 122 | 118 |
| 13 | 153 | 172 |
| 14 | 146 | 148 |
| 15 | 129 | 129 |
| 16 | 121 | 136 |
| 17 | 30 | 31 |

Le tableau ci-dessus montre que les concentrations de CPSE sont sensiblement identiques pour des échantillons de sérum et de plasma héparinisé.

## Revendications

1. Méthode d'analyse permettant le diagnostic *in vitro* d'une hyperplasie bénigne de la prostate (HBP) chez un chien, en utilisant la CPSE contenue dans des échantillons de sérum ou de plasma prélevés sur le chien ainsi que des molécules biologiques fixant spécifiquement la CPSE, **caractérisée en ce que** :
a. on prélève un échantillon de sang chez un chien ;
b. on mesure une concentration en Canine Prostate Specific Esterase (CPSE) dudit échantillon de sérum ou de plasma ;
c. on compare ladite concentration mesurée en CPSE à une valeur de référence, cette valeur étant comprise entre 34,8 ng/ml et 102,4 ng/ml pour le sérum ou le plasma ;
d. on utilise la concentration en CPSE comme marqueur de l'HBP ; et
e. on diagnostique l'HBP chez le chien pour des concentrations de CPSE mesurées dans lesdits échantillons supérieures à ladite valeur de référence.

2. Méthode d'analyse selon la revendication 1, **caractérisée en ce que** la valeur de référence est de 61 ng/ml pour le sérum ou le plasma.

3. Méthode d'analyse selon l'une des revendications 1 à 2, **caractérisée en ce qu'**à l'étape e), on distingue l'HBP de la prostatite et/ou de l'adénocarcinome de la prostate.

4. Méthode d'analyse selon l'une des revendications précédentes, **caractérisée en ce qu'**elle permet le suivi de l'involution de la prostate lors d'un traitement avec des anti-androgènes, et d'adapter ledit traitement en fonction de l'animal.

5. Méthode d'analyse selon l'une des revendications précédentes, **caractérisée en ce que** les molécules biologiques fixant spécifiquement la CPSE utilisées sont un anticorps polyclonal.

6. Méthode d'analyse selon l'une des revendications 1 à 4, **caractérisée en ce que** les molécules biologiques fixant spécifiquement la CPSE utilisées sont un anticorps monoclonal.

7. Méthode d'analyse selon l'une des revendications 1 à 4, **caractérisée en ce que** les molécules biologiques fixant spécifiquement la CPSE utilisées sont des récepteurs solubles ou des aptamères.

8. Méthode d'analyse selon l'une des revendications précédentes, **caractérisée en ce que** la concentration en CPSE est mesurée soit en utilisant la méthode ELISA soit par Immunochromatographie quantitative sur membrane.

9. Utilisation d'un kit de diagnostic in vitro comprenant des molécules biologiques fixant spécifiquement la CPSE pour doser la CPSE contenue dans le sérum ou le plasma du chien destiné à diagnostiquer une HBP chez le chien ayant un taux sérique ou plasmatique de CPSE supérieur à une valeur comprise entre 34,8 ng/ml et 102,4 ng/ml.

10. Utilisation d'un kit de diagnostic in vitro selon la revendication 9, **caractérisée en ce que** l'on diagnostique une HBP chez le chien ayant un taux sérique ou plasmatique de CPSE supérieur à une valeur comprise entre 61 ng/ml et 102,4 ng/ml.

11. Utilisation d'un kit de diagnostic in vitro selon l'une des revendications 9 ou 10, **caractérisée en ce que** les molécules biologiques fixant spécifiquement la CPSE sont des anticorps polyclonaux ou monoclonaux, des récepteurs solubles ou des aptamères.

12. Utilisation d'un kit de diagnostic in vitro selon l'une des revendications 9 à 11, **caractérisée en ce que** les molécules biologiques fixant spécifiquement la CPSE sont couplées à des grosses molécules de type blotine, avidine, streptavidine.

13. Utilisation d'un kit de diagnostic in vitro selon l'une des revendications 9 à 12, **caractérisée en ce que** les molécules biologiques fixant spécifiquement la CPSE sont immobilisées à un support solide.

14. Utilisation d'un kit de diagnostic in vitro selon l'une des revendications 9 à 13, **caractérisée en ce que** les molécules biologiques fixant spécifiquement la CPSE sont immobilisées à des membranes de nitrocellulose ou autres polymères, des supports latex, ou des matériels de plastique.

15. Utilisation d'un kit de diagnostic in vitro selon l'une des revendications 9 à 14, pour le suivi du traitement thérapeutique d'un chien atteint d'une HBP.

## Patentansprüche

1. Analyseverfahren zur *in vitro*-Diagnose einer benignen Hyperplasie der Prostata (BPH) bei einem Hund unter Verwendung der CPSE, die in Serum- oder Plasmaproben enthalten ist, die von einem Hund entnommen wurden, ebenso wie biologischen Molekülen, die spezifisch die CPSE fixieren, **dadurch gekennzeichnet, dass**:
a. eine Blutprobe von einem Hund entnommen wird,
b. eine Konzentration von Canine Prostate Specific Esterase (CPSE) der Serum- oder Plasmaprobe gemessen wird;
c. die gemessene Konzentration von CPSE mit einem Referenzwert verglichen wird, wobei dieser Wert zwischen 34,8 ng/ml und 102,4 ng/ml für das Serum oder das Plasma liegt;
d. die Konzentration von CPSE als Marker der BPH verwendet wird; und
e. die BPH beim Hund für Konzentrationen von CPSE diagnostiziert wird, die in den Proben gemessen werden, die größer als der Referenzwert sind.

2. Analyseverfahren nach Anspruch 1, **dadurch gekennzeichnet, dass** der Referenzwert 61 ng/ml für das Serum oder das Plasma ist.

3. Analyseverfahren nach einem der Ansprüche 1 bis 2, **dadurch gekennzeichnet, dass** im Schritt e) die BPH von Prostatitis und/oder dem Adenokarzinom der Prostata unterschieden wird.

4. Analyseverfahren nach einem der vorhergehenden Ansprüche, **dadurch gekennzeichnet, dass** es die Nachsorge der Involution der Prostata bei einer Behandlung mit Antiandrogenen und die Anpassung der Behandlung je nach dem Tier ermöglicht.

5. Analyseverfahren nach einem der vorhergehenden Ansprüche, **dadurch gekennzeichnet, dass** die verwendeten biologischen Moleküle, die spezifisch die CPSE fixieren, ein polyklonaler Antikörper sind.

6. Analyseverfahren nach einem der Ansprüche 1 bis 4, **dadurch gekennzeichnet, dass** die verwendeten biologischen Moleküle, die spezifisch die CPSE fixieren, ein monoklonaler Antikörper sind.

7. Analyseverfahren nach einem der Ansprüche 1 bis 4, **dadurch gekennzeichnet, dass** die verwendeten biologischen Moleküle, die spezifisch die CPSE fixieren, lösliche Rezeptoren oder Aptemere sind.

8. Verfahren nach einem der vorhergehenden Ansprüche, **dadurch gekennzeichnet, dass** die Konzentration von CPSE entweder unter Verwendung des ELISA-Verfahrens oder durch quantitative Immunchromatographie auf Membran gemessen wird.

9. Verwendung eines *in vitro*-Diagnosekits umfassend biologische Moleküle, die spezifisch die CPSE pro Dosis fixieren, wobei die CPSE, die im Serum oder im Plasma des Hundes enthalten ist, die dazu bestimmt ist, eine BPH beim Hund zu diagnostizieren, einen Serum- oder Plasmaspiegel von CPSE aufweist, der größer als ein Wert ist, der zwischen 34,8 ng/ml und 102,4 ng/ml liegt.

10. Verwendung eines in vitro-Diagnosekits nach Anspruch 9, **dadurch gekennzeichnet, dass** eine BPH beim Hund diagnostiziert wird, die einen Serum- oder Plasmaspiegel von CPSE aufweist, der größer als ein Wert ist, der zwischen 61 ng/ml und 102,4 ng/ml liegt.

11. Verwendung eines *in vitro*-Diagnosekits nach einem der Ansprüche 9 oder 10, **dadurch gekennzeichnet, dass** die biologischen Moleküle, die spezifisch die CPSE fixieren, polyklonale oder monoklonale Antikörper, lösliche Rezeptoren oder Aptemere sind.

12. Verwendung eines *in vitro*-Diagnosekits nach einem der Ansprüche 9 bis 11, **dadurch gekennzeichnet, dass** die biologischen Moleküle, die spezifisch die CPSE fixieren, an große Moleküle vom Typ Biotin, Avidin, Streptavidin gekoppelt sind.

13. Verwendung eines *in vitro*-Diagnosekits nach einem der Ansprüche 9 bis 12, **dadurch gekennzeichnet, dass** die biologischen Moleküle, die spezifisch die CPSE fixieren, an einem festen Träger immobilisiert sind.

14. Verwendung eines *in vitro*-Diagnosekits nach einem der Ansprüche 9 bis 13, **dadurch gekennzeichnet, dass** die biologischen Moleküle, die spezifisch die CPSE fixieren, an Membranen aus Nitrocellulose oder anderen Polymeren, Latexträgern oder Plastikmaterialien immobilisiert sind.

15. Verwendung eines *in vitro*-Diagnosekits nach einem der Ansprüche 9 bis 14 für die Nachsorge der therapeutischen Behandlung eines Hundes, der an BPH leidet.

## Claims

1. Analysis method for the in vitro diagnosis of benign prostatic hyperplasia (BPH) in dogs, using the CPSE contained in serum or plasma samples taken from the dog and biological molecules specifically binding CPSE, **characterised in that**:
a. a blood sample is taken from a dog;
b. a Canine Prostate Specific Esterase (CPSE) concentration of said serum or plasma sample is measured;
c. said measured CPSE concentration is compared to a reference value, this value being between 34.8 ng/ml and 102.4 ng/ml for serum or plasma;
d. the CPSE concentration is used as a marker for BPH; and
e. BPH is diagnosed in dogs for CPSE concentrations measured in said samples greater than said reference value.

2. Analysis method according to claim 1, **characterised in that** the reference value is 61 ng/ml for the serum or plasma.

3. Analysis method according to any of claims 1 to 2, **characterised in that**, in step e), BPH is distinguished from prostatitis and/or prostate adenocarcinoma.

4. Analysis method according to any of the above claims, **characterised in that** it is suitable for the follow-up prostatic involution during anti-androgen treatment, and adapting said treatment according to the animal.

5. Analysis method according to any of the above claims, **characterised in that** the biological molecules specifically binding CPSE used are a polyclonal antibody.

6. Analysis method according to any of claims 1 to 4, **characterised in that** the biological molecules specifically binding CPSE used are a monoclonal antibody.

7. Analysis method according to any of claims 1 to 4, **characterised in that** the biological molecules specifically binding CPSE used are soluble receptors or aptamers.

8. Analysis method according to any of the above claims, **characterised in that** the CPSE concentration is measured either using an ELISA method or by means of membrane-based quantitative immunochromatagraphy.

9. Use of an in vitro diagnostic kit comprising biological molecules specifically binding CPSE for assaying the CPSE contained in dog serum or plasma for diagnosing BPH in dogs having a serum or plasma CPSE level greater than a value between 34.8 ng/ml and 102.4 ng/ml.

10. Use of an in vitro diagnostic kit according to claim 9, **characterised in that** BPH is diagnosed in dogs having a serum or plasma CPSE level greater than a value between 61 ng/ml and 102.4 ng/ml.

11. Use of an in vitro diagnostic kit according to any of claims 9 or 10, **characterised in that** the biological molecules specifically binding CPSE are polyclonal or monoclonal antibodies, soluble receptors or aptamers.

12. Use of an in vitro diagnostic kit according to any of claims 9 to 11, **characterised in that** the biological molecules specifically binding CPSE are coupled with large molecules such as biotin, avidin, streptavidin.

13. Use of an in vitro diagnostic kit according to any of claims 9 to 12, **characterised in that** the biological molecules specifically binding CPSE are fixed to a solid substrate.

14. Use of an in vitro diagnostic kit according to any of claims 9 to 13, characterised that the biological molecules specifically binding CPSE are fixed to membranes of nitrocellulose or other polymers, latex substrates, or plastic materials.

15. Use of an in vitro diagnostic kit according to any of claims 9 to 14, for the follow-up of the therapeutic treatment of dogs suffering from BPH.
